**Europäisches Patentamt**

**European Patent Office**

**Office européen des brevets**

(19)

(11) Veröffentlichungsnummer : **0 305 749 B1**

# EUROPÄISCHE PATENTSCHRIFT

(12)

(45) Veröffentlichungstag der Patentschrift :
18.09.91 Patentblatt 91/38

(51) Int. Cl.⁵ : **C08F 8/34, C12N 11/08**

(21) Anmeldenummer : **88112531.4**

(22) Anmeldetag : **02.08.88**

(54) **Thiolgruppenhaltige Polymere, Verfahren zu ihrer Herstellung und ihre Verwendung.**

(30) Priorität : **08.08.87 DE 3726454**

(43) Veröffentlichungstag der Anmeldung :
**08.03.89 Patentblatt 89/10**

(45) Bekanntmachung des Hinweises auf die
Patenterteilung :
**18.09.91 Patentblatt 91/38**

(84) Benannte Vertragsstaaten :
**AT BE CH DE ES FR GB GR IT LI LU NL SE**

(56) Entgegenhaltungen :
**EP-A- 0 000 486**
**EP-A- 0 124 782**
**FR-A- 2 289 534**
**GB-A- 1 072 658**
**US-A- 3 884 761**
**US-A- 3 900 451**

(73) Patentinhaber : **BEHRINGWERKE**
**Aktiengesellschaft**
**Postfach 1140**
**W-3550 Marburg 1 (DE)**

(72) Erfinder : **Stüber, Werner, Dr.**
**Cölber Weg 12**
**W-3551 Lahntal (DE)**
Erfinder : **Löbermann, Hartmut, Dr.**
**Mooswaldstrasse 7b**
**W-7801 Schallstadt (DE)**

(74) Vertreter : **Klein, Otto, Dr. et al**
**Hoechst AG Zentrale Patentabteilung**
**Postfach 80 03 20**
**W-6230 Frankfurt am Main 80 (DE)**

## Beschreibung

Gegenstand der Erfindung sind thiolgruppenhaltige Polymere, Verfahren zu ihrer Herstellung und ihre Verwendung.

Polymere Trägermaterialien, die mit Mercaptogruppen derivatisiert sind, sind von großem Interesse, da hiermit einerseits Substanzen gereinigt werden, die üblicherweise mit Sulfhydrylgruppen in Wechselwirkung treten und andererseits Substanzen an Sulfhydrylgruppen irreversibel immobilisiert werden können. Solche Substanzen können beispielsweise Enzyme, Antikörper, Proenzyme oder Antigene sein. Als Trägermatrix werden bislang insbesondere Derivate der Agarose eingesetzt. Besondere Beachtung verdient bei der Herstellung dieser polymeren Träger die chemische Einführung der Sulfhydrylfunktionen. Bekannt und damit dem Stand der Technik entsprechend sind Polymerderivatisierungen mit Homocysteinthiolacton, Glutathion-Kupplungen mit vorangegangener Bromcyan-Aktivierung sowie die Aktivierung mit Epichlorhydrin und nachfolgende Reaktion mit Thiosulfat samt einem Reduktionsschritt, als auch eine Oxirangruppenumsetzung mit Dithiothreitol (R. Axén et al., Acta Chemica Scan. B 29, 471-474, 1975).

Diese thiolhaltigen Polymeren sowie deren Herstellungsverfahren sind jedoch mit Nachteilen behaftet und somit verbesserungswürdig. Zum einen liegen die Gründe hierfür in der Matrix, die aufgrund der Kohlenhydratstruktur mechanisch empfindlich und auch mikrobiell angreifbar ist. Zum anderen liegen die Gründe im Verfahren zur Sulfhydrylgruppeneinführung, das entweder teuer oder umständlich ist und bei manchen Verfahren auch die Entstehung instabiler Bindungen beinhaltet.

Die Aufgabe der Erfindung bestand somit darin, ein Verfahren zu entwickeln, das es gestattet, thiolgruppenhaltige Polymere einfach und wirtschaftlich herzustellen. Darüber hinaus mußte die Bedingung erfüllt sein, daß sich die entstehenden chemischen Bindungen durch eine hohe Stabilität auszeichnen.

Aufgabe der Erfindung war auch, thiolgruppenhaltige Polymere herzustellen, die sich aufgrund der zur Derivatisierung eingesetzten Matrix durch eine überlegene mechanische und chemische Stabilität auszeichnen.

Es wurde nun überraschenderweise gefunden, daß Polymere, die mit Oxirangruppen derivatisiert waren, mit wasserlöslichen Salzen des Schwefelwasserstoffes unter Bildung von immobilisierten Sulfhydrylfunktionen reagierten.

Gegenstand der Erfindung ist ein thiolgruppenhaltiges Polymer erhältlich dadurch, daß in ein Hydroxygruppen enthaltendes wasserunlösliches Polymer Oxirangruppen eingeführt werden und das Oxirangruppen enthaltende Polymer mit einem wasserlöslichen Salz des Schwefelwasserstoffs vorzugsweise einem Alkali- oder Erdalkalisalz behandelt wird.

Als Hydroxygruppen enthaltende Polymere sind Polymere auf Kohlenhydratbasis, beispielsweise Agarosegele oder Dextrangele, oder Polymerisate auf Basis von Methacrylamid, N-Methylen-bis-methycrylamid, Glycidylmethacrylat-Polyethylenglykol-Derivaten und Pentaerythritdimethacryl geeignet.

Polymere, die gemäß ihrer chemischen Natur als Copolymerisate bezeichnet werden und aus Acrylsäure- oder Methacrylsäurederivaten aufgebaut und mit Oxirangruppen versehen sind, eignen sich bevorzugt für die Herstellung thiolgruppenhaltiger Polymere. Monomerbestandteile solcher Copolymerisate sind beispielsweise Methacrylamid, M-Methylen-bismethacrylamid, Glycidylmethacrylate, Glycidylmethacrylat-Polyethylenglykolderivate oder Pentaerythritmethacrylsäurederivate. Insbesondere sind Harze geeignet, die unter den Bezeichnungen [R]Fractogel oder [R]Sepabeads im Handel sind.

Die Hydroxygruppen dieser wasserunlöslichen Harze werden nach bekannten Methoden z.B. nach EP-A-0203463 mit 1-Chlor-2,3-epoxypropan (Epichlorhydrin) oder mit 1,4-bis(2,3-epoxypropoxy)butan umgesetzt und somit Oxirangruppen in die Polymere eingeführt.

Die Epoxidierung erfolgt beispielsweise so, daß 1 kg wasserfeuchtes Polymer in 1500 ml Wasser und 650 ml 2 N Natronlauge suspendiert wird und mit 200 ml Epichlorhydrin bei erhöhter Temperatur, beispielsweise 40-50°C, zur Reaktion gebracht wird. Nach einem Zeitraum von 1-4 Stunden wird das Polymer mit Wasser gewaschen und trockengesaugt.

Das oxiranhaltige Polymer wird dann in Wasser oder einer wässrigen Pufferlösung aufgenommen, wobei üblicherweise pro kg Harz 500 ml bis 3 l, bevorzugt jedoch etwa 1 l zugesetzt werden. Als Puffer können Salzlösungen, beispielsweise Natriumphosphatpuffer, Natriumcitratpuffer oder Hepespuffer mit einem pH von 4 bis pH 13 eingesetzt werden. Bevorzugt wird pro kg Polymer 1 Liter einer 0,25 molaren Natriumhydrogencarbonatlösung zugesetzt.

Daraufhin werden 10-200 g eines wasserlöslichen Sulfids oder Hydrogensulfids, bevorzugt eines Alkalisulfids zugesetzt. Besonders bevorzugt werden 70-80 g Natriumsulfid $\times$ 9$H_2$O zugesetzt und der Ansatz bei einer Temperatur von 0-70°C, bevorzugt bei 35-45°C gerührt. Als Reaktionszeit wird 2 bis 48 Stunden, bevorzugt 12-14 Stunden gewählt. Das Polymer wird dann mit Wasser oder/und einer 0,5 ml/100 ml Essigsäure gewaschen.

Gegenstand der Erfindung ist auch ein Verfahren zur Herstellung eines thiolgruppenhaltigen Polymers, dadurch gekennzeichnet, daß ein Hydroxygruppen enthaltendes wasserunlösliches Polymer bei 40-50°C eine bis vier Stunden mit 1-Chlor-2,3-epoxypropan (Epichlorhydrin) oder 1,4-bis(2,3-epoxypropoxy)butan umgesetzt wird, wodurch Oxirangruppen in das Polymer eingeführt werden, das Polymer mit Wasser gewaschen, in wässriger Lösung aufgenommen, mit einem wasserlöslichen Sulfid bei einer Temperatur von 0-70°C 2 bis 48 Stunden behandelt und das Polymer mit Wasser oder verdünnter Säure gewaschen wird.

Bevorzugt ist ein erfindungsgemäßes Polymeres, das erhalten werden kann, indem in ein Copolymerisat aus Pentaerythrit, Methacrylsäurederivaten und Polyethylenglykol, das mit Divinylbenzol vernetzt ist, Oxirangruppen durch Reaktion mit Epichlorhydrin eingeführt werden und mit Natriumsulfid behandelt wird.

Weiterhin ist bevorzugt ein erfindungsgemäßes Polymeres, das erhalten werden kann, indem in ein Polymer auf der Basis von Methacrylsäurederivaten (RSepabeads), die Oxirangruppen durch Reaktion mit Butandioldiglycidylether eingeführt werden und mit Natriumsulfid behandelt wird.

Die nach obigem Verfahren thiolfunktionisierten polymeren Träger eignen sich aufgrund ihrer mechanischen und chemischen Stabilität ganz besonders bevorzugt zur Immobilisierung von Enzymen, Proenzymen, Antikörpern oder Antigenen sowie zur Chromatographie von Substanzen, die mit Mercaptogruppen in Wechselwirkung treten.

Ferner eignen sich die so hergestellten thiolhaltigen Polymere, um nach vorangegangener Aktivierung mit einem Disulfid mit thiolgruppenhaltigen Substanzen, zum Beispiel Proteinen, unter Disulfidaustausch in Reaktion zu treten. Dadurch lassen sich solche Proteine konzentrieren oder vom Polymer eluieren und somit reinigen. Disulfide, die zur Aktivierung der polymeren Mercaptogruppen besonders geeignet sind, sind beispielsweise 2,2'-Dipyridyldisulfid, Bis-(2-pyridyl-N-oxid)-disulfid oder 5,5'-Dithiobis-(2-nitrobenzoesäure).

Bevorzugt kann ein erfindungsgemäßes Polymeres zur Reinigung von alpha$_1$-Antitrypsin verwendet werden.

Die folgenden Beispiele erläutern die Erfindung.

Beispiel 1

Herstellung eines thiolhaltigen Polymers auf RFractogel-Basis

1000 ml RFractogel HW-65 (F), ein Copolymerisat aus Pentaerythrit, Methacrylsäurederivaten und Polyethylenglykol (Hersteller : Toyo Soda) wurden mit 350 ml Wasser und 275 ml 5 N Natronlauge versetzt und auf 45°C erwärmt. Es wurden 200 ml Epichlorhydrin zugesetzt und 2 Stunden bei dieser Temperatur gerührt. Es wurde abfiltriert und mit Wasser so lange gewaschen, bis der pH-Wert auf 7 gefallen war. Das trockengesaugte Harz wurde in 1400 ml 0,2 mol/l Natriumhydrogencarbonatlösung aufgenommen und mit 72 g Na$_2$S × 9H$_2$O versetzt. Der Ansatz wurde 15 Stunden bei 40°C gerührt, abgesaugt, mit Wasser und anschließend mit 0,5 ml/100 ml Essigsäure ausgewaschen.

Beispiel 2

Herstellung eines thiolhaltigen Polymers auf RSepabeads-Basis (Hersteller : Mitsubishi)

1000 ml RSepabeads HG05 wurden in 1000 ml 0,6 N NaOH, die 2 g NaBH$_4$, enthielt, suspendiert. Anschließend wurden 1000 ml Butandioldiglycidylether zugesetzt und die Suspension 8 Stunden bei Raumtemperatur gerührt. Das epoxiaktivierte RSepabeads HG05-Material wurde anschließend auf einer Glasfritte mit 15 l Wasser gewaschen. Die Umsetzung mit Na$_2$S × 9H$_2$O erfolgt wie in Beispiel 1 beschrieben.

Beispiel 3

Aktivierung von thiolhaltigem RFractogel mit 2,2'-Dithiobis-pyridin-N-oxid

500 ml thiolhaltiges RFractogel HW-65, das wie in Beispiel 1 beschrieben hergestellt wurde, wurde mit einem Puffer A, der 50 mmol/l Tris/HCl und 150 mmol/l NaCl, pH 7.5 enthielt, äquilibriert. Anschließend wurde das Harzmaterial 4 × mit je 1 l Puffer A + 1,5 mmol/l 2,2'-Dithiobis-pyridin-N-oxid für 320 min bei Raumtemperatur inkubiert und danach wieder in Puffer A gewaschen. Das so aktivierte Harzmaterial kann nun beispielsweise für Disulfidaustauschreaktionen verwendet werden.

Beispiel 4

Aktivierung von thiolhaltigem RSepabeads HG05 mit Di-(2-pyridyl)disulfid

500 ml thiolhaltige RSepabeads HG05, die wie in Beispiel 2 beschrieben hergestellt wurden, wurden mit 1,5 mmol/l Di-(2-pyridyl)-disulfid unter den Bedingungen von Beispiel 3 aktiviert.

Beispiel 5

Reinigung von alpha$_1$-Antitrypsin

500 ml Human-Plasma wurde mit 1000 ml Wasser, das 30 mmol/l Mercaptoethanol enthielt, verdünnt, 1 Stunde bei Raumtemperatur inkubiert und anschließend einer fraktionierten Ammoniumsulfatfällung zwischen 50% Sättigung (w/v) und 75% (w/v) Sättigung unterworfen. Der 50%-75% (w/v) Rückstand wurde in einer Lösung, die 50 mmol/l Tris, 200 mmol/l NaCl und 1 mmol/l EDTA, pH 8,0 enthielt, gelöst und über eine 500 ml RACA 202-Säule chroma-

tographiert. Die alpha₁-Antitrypsin enthaltenden Fraktionen wurden vereinigt und auf eine mit 2,2'-Dithiobis-(pyridin)-N-oxid aktivierte Thiol-RFractogel- Säule (100 ml) gegeben. Die Säule wurde mit dem gleichen Puffer gewaschen und alpha₁-Antitrypsin mittels einer Lösung, die 10 mmol/l Dithiothreitol enthielt, eluiert. Das so gewonne alpha₁-Antitrypsin war weitestgehend von Verunreinigungen befreit.

**Patentansprüche**

**Patentansprüche für folgende Vertragsstaaten : AT, BE, DE, CH, FR, GB, IT, LI, LU, NL, SE**

1. Thiolgruppenhaltiges Polymer erhältlich dadurch, daß in ein Hydroxygruppen enthaltendes wasserunlösliches Polymer Oxirangruppen eingeführt werden und das Oxirangruppen enthaltende Polymer mit einem wasserlöslichen Salz des Schwefelwasserstoffs behandelt wird.

2. Polymer nach Anspruch 1, dadurch gekennzeichnet, daß das Hydroxygruppen enthaltende Polymer ein Polymer auf Kohlenhydratbasis, ein Polymerisat auf Basis von Methacrylamid, N-Methylen-bis-methacrylamid, Glycidylmethacrylat-Polyethylenglykol-Derivaten oder Pentaerythritdimethacrylat ist.

3. Polymer nach Anspruch 1, dadurch gekennzeichnet, daß das Hydroxygruppen enthaltende Polymer ein Agarosegel ist.

4. Polymer nach Anspruch 1, dadurch gekennzeichnet, daß das Salz des Schwefelwasserstoffs ein Alkalisalz oder Erdalkalisalz ist.

5. Polymer nach Anspruch 1, dadurch gekennzeichnet, daß das Oxirangruppen enthaltende Polymer Acryl- oder Methacrylsäurederivate enthält.

6. Polymer nach Anspruch 1, dadurch gekennzeichnet, daß das Hydroxygruppen enthaltende wasserunlösliche Polymer ein Copolymerisat aus Pentaerythrit, Methacrylsäurederivaten und Polyethylenglykol ist, das mit Divinylbenzol vernetzt ist, daß die Oxirangruppen durch Reaktion dieses Copolymerisats mit Epichlorhydrin eingeführt werden, und daß mit Natriumsulfid behandelt wird.

7. Polymer nach Anspruch 1, dadurch gekennzeichnet, daß das Hydroxygruppen enthaltende wasserunlösliche Polymer ein Polymer auf der Basis von Methacrylsäurederivaten (RSepabeads) ist, daß die Oxirangruppen durch Reaktion dieses Polymers mit Butandioldiglycidylether eingeführt werden, und daß mit Natriumsulfid behandelt wird.

8. Verfahren zur Herstellung eines thiolgruppenhaltigen Polymers, dadurch gekennzeichnet, daß ein Hydroxygruppen enthaltendes wasserunlösliches Polymer bei 40-50°C eine bis vier Stunden mit 1-Chlor-2,3-epoxypropan (Epichlorhydrin) oder 1,4-bis(2,3-epoxypropoxy)butan umgesetzt wird, wodurch Oxirangruppen in das Polymer eingeführt werden, das Polymer mit Wasser gewaschen, in wässriger Lösung aufgenommen, mit einem wasserlöslichen Sulfid bei einer Temperatur von 0-70°C 2 bis 48 Stunden behandelt und das Polymer mit Wasser oder verdünnter Säure gewaschen wird.

9. Verwendung eines Polymers nach Anspruch 1, zur Immobilisierung von Enzymen, Proenzymen, Antikörpern oder Antigenen sowie zur Chromatographie von Substanzen, die mit Mercaptogruppen in Wechselwirkung treten.

10. Verwendung eines Polymers nach Anspruch 1 nach Aktivierung mit einem Disulfid zur Bindung von thiolgruppenhaltigen Substanzen.

11. Verwendung eines Polymeren nach Anspruch 1 zur Reinigung von alpha₁-Antitrypsin.

**Patentansprüche für folgende Vertragsstaaten : ES, GR**

1. Verfahren zur Herstellung eines thiolgruppenhaltigen Polymers, dadurch gekennzeichnet, daß ein Hydroxygruppen enthaltendes wasserunlösliches Polymer bei 40-50°C eine bis vier Stunden mit 1-Chlor-2,3-epoxypropan (Epichlorhydrin) oder 1,4-bis (2,3-epoxypropoxy) butan umgesetzt wird, wodurch Oxirangruppen in das Polymer eingeführt werden, das Polymer mit Wasser gewaschen, in wässrige Lösung aufgenommen, mit einem wasserlöslichen Sulfid bei einer Temperatur von 0-70°C 2 bis 48 Stunden behandelt und das Polymer mit Wasser oder verdünnter Säure gewaschen wird.

2. Verfahren nach Anspruch 1, dadurch gekennzeichnet, daß das Hydroxygruppen enthaltende Polymer ein Polymer auf Kohlenhydratbasis, ein Poylmerisat auf Basis von Methacrylamid, N-Methylen-bis-methacrylamid, Glycidylmethacrylat-Polyethylenglykol-Derivaten oder Pentaerythritidimethacrylat ist.

3. Verfahren nach Anspruch 1, dadurch gekennzeichnet, daß das Hydroxygruppen enthaltende Polymer ein Agarosegel ist.

4. Verfahren nach Anspruch 1, dadurch gekennzeichnet, daß das Salz des Schwefelwasserstoffs ein Alkalisalz oder Erdalkalisalz ist.

5. Verfahren nach Anspruch 1, dadurch gekennzeichnet, daß das Oxirangruppen enthaltende Polymer Acryl- oder Methacrylsäurederivate enthält.

6. Verfahren nach Anspruch 1, dadurch gekennzeichnet, daß das Hydroxygruppen enthaltende wasserunlösliche Polymer ein Copolymerisat aus Pentaerythrit, Methacrylsäurederivaten und Polyethylenglykol ist, das mit Divinylbenzol vernetzt ist, daß die Oxirangruppen durch Reaktion dieses Copolymerisats mit Epichlorhydrin eingeführt werden, und daß mit Natriumsulfid behandelt wird.

7. Polymer nach Anspruch 1, dadurch gekennzeichnet, daß das Hydroxygruppen enthaltende wasserunlösliche Polymer ein Polymer auf der Basis von Methacrylsäurederivaten (RSepabeads) ist, daß die Oxirangruppen durch Reaktion dieses Polymers mit Butandioldiglycidylether eingeführt werden, und daß mit Natriumsulfid behandelt wird.

## Claims

**Claims for the following Contracting States : AT, BE, DE, CH, FR, GB, IT, LI, LU, NL, SE**

1. A polymer containing thiol groups which is obtainable by introducing oxirane groups into a water-insoluble polymer containing hydroxyl groups and treating the polymer containing oxirane groups with a water-soluble salt of hydrogen sulfide.

2. A polymer as claimed in claim 1, wherein the polymer containing hydroxyl groups is a carbohydrate-based polymer or a polymer based on methacrylamide, N-methylene-bis-methacrylamide, glycidyl methacrylate/polyethylene glycol derivatives or pentaerythritol dimethacrylate.

3. A polymer as claimed in claim 1, wherein the polymer containing hydroxyl groups is an agarose gel.

4. A polymer as claimed in claim 1, wherein the salt of hydrogen sulfide is an alkali metal salt or an alkaline earth metal salt.

5. A polymer as claimed in claim 1, wherein the polymer containing oxirane groups contains acrylic acid derivatives or methacrylic acid derivatives.

6. A polymer as claimed in claim 1, wherein the water-insoluble polymer containing hydroxyl groups is a copolymer prepared from pentaerythritol, methacrylic acid derivatives and polyethylene glycol and which is crosslinked with divinylbenzene, wherein the oxirane groups are introduced by reacting this copolymer with epichlorohydrin, and wherein it is treated with sodium sulfide.

7. A polymer as claimed in claim 1, wherein the water-insoluble polymer containing hydroxyl groups is a polymer based on methacrylic acid derivatives (RSepabeads), wherein the oxirane groups are introduced by reacting this polymer with butanediol diglycidyl ether, and wherein it is treated with sodium sulfide.

8. A process for the preparation of a polymer containing thiol groups, which comprises reacting a water-insoluble polymer containing hydroxyl groups for one to four hours at 40-50°C with 1-chloro-2,3-epoxypropane (epichlorohydrin) or 1,4-bis(2,3-epoxy-propoxy)butane, whereby oxirane groups are introduced into the polymer, washing the polymer with water, taking it up in aqueous solution and treating it for 2 to 48 hours at a temperature of 0-70°C with a water-soluble sulfide, and washing the polymer with water or dilute acid.

9. The use of a polymer as claimed in claim 1 for immobilization of enzymes, proenzymes, antibodies or antigens and for chromatography of substances which interact with mercapto groups.

10. The use of a polymer as claimed in claim 1, after activation using a disulfide, for binding substances containing thiol groups.

11. The use of a polymer as claimed in claim 1 for purification of alpha$_1$-antitrypsin.

**Claims for the following Contracting States : ES, GR**

1. A process for the preparation of a polymer containing thiol groups, which comprises reacting a water-insoluble polymer containing hydroxyl groups for one to four hours at 40-50°C with 1-chloro-2,3-epoxypropane (epichlorohydrin) or 1,4-bis(2,3-epoxy-propoxy)butane, whereby oxirane groups are introduced into the polymer, washing the polymer with water, taking it up in aqueous solution and treating it for 2 to 48 hours at a temperature of 0-70°C with a water-soluble sulfide, and washing the polymer with water or dilute acid.

2. The process as claimed in claim 1, wherein the polymer containing hydroxyl groups is a carbohydrate-based polymer or a polymer based on methacrylamide, N-methylene-bis-methacrylamide, glycidyl methacrylate/polyethylene glycol derivatives or pentaerythritol dimethacrylate.

3. The process as claimed in claim 1, wherein the polymer containing hydroxyl groups is an agarose gel.

4. The process as claimed in claim 1, wherein the salt of hydrogen sulfide is an alkali metal salt or an alkaline earth metal salt.

5. The process as claimed in claim 1, wherein the polymer containing oxirane groups contains acrylic acid derivatives or methacrylic acid derivatives.

6. The process as claimed in claim 1, wherein the water-insoluble polymer containing hydroxyl groups is a copolymer prepared from pentaerythritol, methacrylic acid derivatives and polyethylene glycol and which is crosslinked with divinylbenzene, wherein the oxirane groups are introduced by reacting this copolymer with epichlorohydrin, and wherein it is treated with sodium sulfide.

7. The process as claimed in claim 1, wherein the water-insoluble polymer containing hydroxyl groups is a polymer based on methacrylic acid derivatives (RSepabeads), wherein the oxirane groups are introduced by reacting this polymer with butanediol diglycidyl ether, and wherein it is treated with sodium sulfide.

## Revendications

### Revendications pour les Etats Contractants suivants : AT, BE, DE, CH, FR, E, IT, LI, LU, NL, SE

1. Polymère porteur de groupes thiol, que l'on peut obtenir par introduction de groupes oxiranne dans un polymère insoluble dans l'eau contenant des groupes hydroxy et traitement du polymère contenant des groupes oxiranne avec un sel soluble dans l'eau de l'acide sulfhydrique.

2. Polymère selon la revendication 1, caractérisé en ce que le polymère contenant des groupes hydroxy est un polymère à base de glucides, un polymérisat à base de méthacrylamide, de N-méthylène-bis-méthacrylamide, de dérivés de méthacrylate de glycidyle-polyéthylèneglycol ou de diméthacrylate de pentaérythrytol.

3. Polymère selon la revendication 1, caractérisé en ce que le polymère contenant des groupes hydroxy est un gel d'agarose.

4. Polymère selon la revendication 1, caractérisé en ce que le sel de l'acide sulfhydrique est un sel alcalin ou un sel alcalino-terreux.

5. Polymère selon la revendication 1, caractérisé en ce que le polymère contenant des groupes oxiranne contient des dérivés acryliques ou méthacryliques.

6. Polymère selon la revendication 1, caractérisé en ce que le polymère insoluble dans l'eau contenant des groupes hydroxy est un copolymérisat de pentaérythrytol, de dérivés méthacryliques et de polyéthylène glycol, réticulé par le divinylbenzène, en ce que les groupes oxiranne sont introduits par réaction de ce copolymérisat avec l'épichlorhydrine et en ce qu'il est traité avec le sulfure de sodium.

7. Polymère selon la revendication 1, caractérisé en ce que le polymère insoluble dans l'eau contenant des groupes hydroxy est un polymère à base de dérivés méthacryliques (RSepabeads), que les groupes oxiranne sont introduits par réaction de ce polymère avec l'éther diglycidylique de butanediol et en ce qu'il est traité avec le sulfure de sodium.

8. Procédé pour préparer un polymère porteur de groupes thiols, caractérisé en ce que l'on fait réagir un polymère insoluble dans l'eau contenant des groupes hydroxy à 40-50°C, pendant une à quatre heures, avec le 1-chloro-2,3-époxypropane (épichlorhydrine) ou le 1,4-bis(2,3-époxypropoxy)-butane, introduisant ainsi des groupes oxiranne dans le polymère, on lave le polymère à l'eau, on le reprend en solution aqueuse, on le traite avec un sulfure soluble dans l'eau à une température de 0 à 70°C pendant 2 à 48 heures et on lave le polymère à l'eau ou avec un acide dilué.

9. Utilisation d'un polymère selon la revendication 1 pour immobiliser des enzymes, des proenzymes, des anticorps ou des antigènes, et pour la chromatographie de substances qui entrent en interaction avec des groupes mercapto.

10. Utilisation d'un polymère selon la revendication 1 après activation avec un disulfure pour fixer des substances porteuses de groupes thiol.

11. Utilisation d'un polymère selon la revendication 1 pour purifier l'alpha$_1$-antitrypsine.

### Revendications pour les Etats Contractants suivants : ES, GR

1. Procédé pour préparer un polymère porteur de groupes thiols, caractérisé en ce que l'on fait réagir un polymère insoluble dans l'eau contenant des groupes hydroxy à 40-50°C, pendant une à quatre heures, avec le 1-chloro-2,3-époxypropane (épichlorhydrine) ou le 1,4-bis(2,3-époxypropoxy)-butane, introduisant ainsi des groupes oxiranne dans le polymère, on lave le polymère à l'eau, on le reprend en solution aqueuse, on le traite avec un sulfure soluble dans l'eau à une température de 0 à 70°C pendant 2 à 48 heures et on lave le polymère à l'eau ou avec un acide dilué.

2. Procédé selon la revendication 1, caractérisé en ce que le polymère contenant des groupes hydroxy est un polymère à base de glucides, un polymérisat à base de méthacrylamide, de N-méthylène-bis-méthacrylamide, de dérivés de méthacrylate de glycidyle-polyéthylèneglycol ou de diméthacrylate de pentaérythrytol.

3. Procédé selon la revendication 1, caractérisé en ce que le polymère contenant des groupes hydroxy est un gel d'agarose.

4. Procédé selon la revendication 1, caractérisé en ce que le sel de l'acide sulfhydrique est un sel alcalin ou un sel alcalino-terreux.

5. Procédé selon la revendication 1, caractérisé en ce que le polymère contenant des groupes oxiranne contient des dérivés acryliques ou méthacryliques.

6. Procédé selon la revendication 1, caractérisé en ce que le polymère insoluble dans l'eau contenant des groupes hydroxy est un copolymérisat de pentaérythrytol, de dérivés méthacryliques et de polyéthylèneglycol, réticulé par le divinylbenzène, en ce que l'on introduit des groupes oxiranne par réaction de ce copolymérisat avec l'épichlorhydrine et en ce que l'on traite avec le sulfure de sodium.

7. Procédé selon la revendication 1, caractérisé en ce que le polymère insoluble dans l'eau contenant des groupes hydroxy est un polymère à base de dérivés méthacryliques (RSepabeads), en ce que l'on introduit des groupes oxiranne par réaction de ce polymère avec l'éther diglycidylique de butanediol et en ce que l'on traite avec le sulfure de sodium.